# EUROPEAN PATENT APPLICATION

(11) **EP 1 550 447 A1**
(43) Date of publication of application: **06.07.2005**
(21) Application number: 03030043.8
(22) Date of filing: 02.01.2004
(51) Int. Cl.: A61K 31/565, A61P 15/00, A61K 31/567, A61K 31/57, A61K 31/585

(54) **Menstrual cycle control and improvement of conception rates in females**

(71) Applicant: SCHERING AKTIENGESELLSCHAFT, 13353 Berlin (DE)
(72) Inventor: Endrikat, Jan, 13125 Berlin (DE); Düsterberg, Bernd, 16727 Bärenklau (DE); Wessel, Jens, 10717 Berlin (DE); Schellschmidt, Ilka, 10825 Berlin (DE)

(57) **Abstract**

The present invention relates to methods of increasing likelihood of conception, treatment of low fecundity and/or providing menstrual cycle control without suppressing ovulation in a female comprising administering an effective dose of at least one estrogen and at least one progestin within a treatment period of at least 21 days to 35 days or multiple periods thereof. Furthermore, the invention relates to the combination of at least one estrogen and at least one progestin for the preparation of a medicament to be used in the abovementioned indications.

A pharmaceutical kit, according to the invention, comprises 21 to 35 dosage units or a multiple of 21 to 35 dosage units, wherein
- a first section comprises at most 21 dosage units of at least one estrogen; and
- a second section comprises from 2 to 34 dosage units of at least one estrogen and from 2 to 34 dosage units of at least one progestin or comprises from 2 to 34 dosage units of at least one estrogen and at least one progestin provided that in each dosage unit containing a progestin or a progestin and an estrogen the progestin is in a non-ovulation inhibiting amount; and
   ; and
- optionally a third section comprises at most 10 dosages of a pharmaceutically acceptable placebo or a blank.

## Description

### FIELD OF INVENTION

The present invention relates to dosing regimens of a combination of an estrogen and a progestin so as to provide menstrual cycle control in a female. Furthermore, the dosing regimens can be used for improving the conception rates in females, in particularly in females with low fecundity.

### BACKGROUND OF INVENTION

More and more couples experience difficulties in getting pregnant, and for a great number of couples the cause cannot be elucidated as sperm insufficiency, tubal impermeation, anovulation or endometriosis. Thus in these couples, the physiology of both the woman and man seems normal and the infertility is unexplained or of sub-infertile nature.

When a couple enters a specialised unit for infertility such as unexplained infertility, extensive examinations of the man and woman are initiated for elucidating the cause of infertility and for specifying a suitable therapy. However, all those examinations and therapeutic treatments induce a lot of stress to the couple resulting in further reduction of the likelihood of conception. Moreover, elucidation of infertility and subsequent treatment often takes several years, which further contradicts the probability of getting pregnant in that fertility usually declines with age. However, it is well known that occasionally the woman gets spontaneously pregnant after having given up all the therapeutic treatments and all the attentions related to that.

There is evidence for that infertility is of multiple factorial nature and not completely understood, such as unexplained infertility or sub-infertility. For example, it is recognised that a number of parameters affect the conception, such as insufficient mucus known as the cervical factor, incomplete follicle ripening, corpus luteum insufficiency, insufficient proliferation of the endometrium and transformation, and psychological issues. It is thought that lack of synchronisation of follicle ripening and endometrial growth can be a potential cause of unexplained infertility or sub-infertility.

Infertility is today treated medically with ovarian stimulating medicaments, such as clomifene, gonatropin stimulating medicaments and ovulation inducing (ovum-releasing) medicaments.

However, there exists no medication directed to the complexed multiple factorial nature of infertility such as unexplained infertility that may be related to poor synchronisation of follicle maturation and endometrial growth. For example, females with poor menstrual cycle control may experience poor synchronisation of follicle maturation and endometrial growth.

The present inventors have found that the administration of a combination of estrogenic and progestinic compounds according to a particular dosing scheme to females lead to better menstrual cycle control. For females trying to be pregnant, the probability of getting pregnant increases as well. Specifically, the present inventors propose that when there is no shortage of time, a female in desire of getting pregnant may benefit from an unspecific initial 1-year treatment period with a proper combination of estrogenic and progestinic compounds as a first step in the treatment of infertility, such as unexplained infertility or sub-fertility.

### SUMMARY OF THE INVENTION

The present invention relates to methods of increasing likelihood of conception, treatment of low fecundity and/or providing menstrual cycle control without suppressing ovulation in a female comprising administering an effective dose of at least one estrogen and at least one progestin within a treatment period of at least 21 days to 35 days or multiple periods thereof. Furthermore, the invention relates to the combination of at least one estrogen and at least one progestin for the preparation of a medicament for increasing likelihood of conception in a female, such as treatment of low fecundity, and/or providing menstrual cycle control.

In a further important aspect the invention relates to a pharmaceutical kit comprising 21 to 35 dosage units or a multiple of 21 to 35 dosage units, wherein within each multiple of 21 to 35 dosage units
- a first section comprises at most 21 dosage units of at least one estrogen; and
- a second section comprises from 2 to 34 dosage units of at least one estrogen and from 2 to 34 dosage units of at least one progestin or comprises from 2 to 34 dosage units of at least one estrogen and at least one progestin provided that in each dosage unit containing a progestin or a progestin and an estrogen the progestin is in a non-ovulation inhibiting amount; and
- optionally a third section comprises at most 21 dosages of a pharmaceutically acceptable placebo or a blank.

### DETAILED DESCRIPTION OF THE INVENTION

It is an object of the present invention to provide methods and products for facilitating fertility and improving conception rates in females. The present inventors have found that when administering combinations of estrogens and progestins in physiological relevant levels and patterns, the conception rate increases, at least in females with low fecundity. It is thought that the beneficial actions of estrogen, such as facilitating sperm ascension through the cervical mucus, induction of endometrial proliferation, providing of positive feed back on follicle growth, providing of estrogen peak levels of about 150 to 200 pg/ml, induction of physiological secretorial transformation of the endometrium for proper nidation of eggs in combination with the beneficial actions of progestins in ripening of eggs could improve the conception rates in women. In summary, the combination of an estrogen and a progestin synchronises follicle ripening and endometrial growth resulting in increased likelihood of conception in a female.

According to the invention, a female should administer the hormones in a manner duplicating the physiological hormonal patterns so as to reduce the variability in menstrual cycle length that may be seen in females with unexplained infertility such as females with low fecundity. Of importance the combination of the estrogen and the progestin may not lead to inhibition of ovulation or suppression of the maturation of eggs.

The present inventors have found that the hormones should be administered in at least two phases followed by one phase without administering any of the hormones administered in the first two phases. The first phase should include administering estrogen only, the second phase should include administering a combination of an estrogen and a progestin and the third phase should include no administering of either progestin or estrogen. Optionally, an estrogen might be administered as the sole active ingredient in a phase intervening between phase two and three. Suitable, phase two might be divided in two sub-phases, IIa and IIb, with different doses of the progestin.Specifically, it is further contemplated that the dose of the progestin in sub-phase IIb is 1.5 to 3 times higher than the dose of the progestin in sub-phase IIa.

According to one embodiment of the invention it is also contemplated that he doses of the estrogen in the two sub-phases IIa and IIb are different, preferably the dose of the estrogen is higher in subphase IIb than in subphase IIa.

Accordingly, an important aspect of the invention relates to a method for increasing likelihood of conception in a female comprising administering to a female, in a treatment period of at least 21 days to 35 days or multiple treatment periods thereof, an effective dose of at least one estrogen and at least one progestin. Another important aspect relates to the use of a combination of at least one estrogen and at least one progestin for the preparation of a medicament for increasing likelihood of conception in a female.

The term "treatment period" is denoted to characterise a period of 21 days to 35 days, preferably 25 to 35 days, more preferably 28 days, wherein the administering of hormones is initiated at day one and wherein a new treatment period may start after 21 to 35 days such as preferably after 28 days. Subsequent treatment periods may follow immediately upon termination of a previous treatment period such that a treatment period may be multiples of 21 to 35 days, such as a multiples of 28 days.

In further aspects the invention relates to methods and uses for the treatment of low fecundity in a female. Furthermore, the beneficial effect of administering a combination of an estrogen and a progestin to a female in a manner according to the present invention may regulate the menstrual cycle so as to achieve cycles of replicate lengths within the normal ranges, such as 25 to 35 days, preferably about 27 to 30 days. Furthermore, the methods and uses may synchronise endometrial proliferation and follicle ripening. Therefore, in still other aspects the methods and uses of the invention include providing menstrual cycle control in a female without suppressing ovulation.

The term "female" is intended to mean any female of mammalian origin, preferably a human female that has experienced menses at least once. The female may be of any age within her fertile age, such as in the age ranging from about 18 to 60 years. As a matter of fact, the fertility of a woman decreases with age for which reason that a female in the present context is in the age ranging from about 25 to 55 years.

However, as recognised by the present inventors the methods and uses of the present invention, such as improving the conception rate in a female, is particularly useful for females in the age ranging from 30 to 50 years, such as from 35 to 50 years. In general, a female may be over 30 years of age, preferably over 35 years of age as long as the female has not experienced menopause yet.

Obviously, methods and uses of the invention is directed to females in a low fecundity category, such as females selected from the group comprising of a female over 30 years of age; a female previously on the birth control pills; a female with irregular menstrual cycle or any other females who typically have lower fecundity, for example females with polycystic ovary syndrome or endometriosis.

In a further aspect, the invention relates to a pharmaceutical kit for use in the methods and uses of the invention. A pharmaceutical kit, according to the invention, comprises 21 to 35 dosage units or a multiple of 21 to 35 dosage units, wherein
- a first section comprises at most 21 dosage units of at least one estrogen; and
- a second section comprises from 2 to 34 dosage units of at least one estrogen and from 2 to 34 dosage units of at least one progestin or comprises from 2 to 34 dosage units of at least one estrogen and at least one progestin provided that in each dosage unit containing a progestin or a progestin and an estrogen the progestin is in a non-ovulation inhibiting amount; and ; and
- optionally a third section comprises at most 10 dosages of a pharmaceutically acceptable placebo or a blank.

### Hormones

In principle, any estrogen and progestin is applicable for use in methods, uses, kits and compositions of the invention as long as the hormones are administered in a manner duplicating physiological levels and physiological fluctuations during the normal menstrual cycle of a female in the fertile age without leading to inhibition of ovulation.

The term "estrogen" is denoted to mean any exogenous substance with estrogenic activity or any substance that may cause release of estrogens in vivo. The term *estrogenic activity* is intended to imply the characteristics of substances exhibiting estrogen-like activities as defined by *in vitro* receptor-binding assays, transactivation assays, or *in vivo* assays as follows:

Receptor binding assays, tranactivation assays, and *in vivo* assays for establishing estrogenic activity are described in *Handbook of experimental Pharmacology, Vol. 135*/*II, Estrogens and Antiestrogens II, Pharmacology and Clinical Applications of Estrogens and Antiestrogens;* M. Ottel and E. Schillinger (editors); K.-H. Fritzemeier and C. Hegele-Hartung, *In Vitro and In Vivo Models to Characterise Estrogens and Antiestrogens;* Springer-Verlag, Berlin, Heidelberg, 1999.

The basic principle behind receptor binding assays lies in that the binding of substances to the estrogen receptor is a pre-requisite for the compound to exhibit estrogen-like activity. Binding affinities or dissociation constants are used as measures of the substance to bind to the estrogen receptor.

Transactivation assays for the detection of estrogenic activity are based on the ability of the estrogen receptor to cause gene activation in a ligand-dependant way. The binding of an estrogenic substance results in ligand-activated formation of a receptor dimer, which then binds to a specific nucleotide sequence in the promoter segment of a target gene.

The term "progestin" is synonymous with the terms gestagen or progestogen, and comprises a class of hormones naturally present in the body as well as synthetic and semi-synthetic derivatives thereof. A progestin is characterised by having progestinic activity as defined by *in vitro* receptor-binding assays, transactivation assays, or *in vivo* assays. The skilled person knows those assays.

As stated at least one estrogen should be administered. The at least one estrogen may be a steroidal or a non-steroidal type of estrogen, that may be further classified as natural or as synthetic estrogen. Furthermore, the at least one estrogen may be an estrogen-receptor agonists such as an ER-α agonist and/or an ER-β agonist. A natural estrogen is defined as an estrogen that is found in plant and animal species such as estrone, estradiol, estriole or derivatives thereof, which are found in humans and other animal species, and equilin or derivatives thereof, which are found in the urine of gravide horses. Natural non-steroidal estrogens include the plant estrogens, phytoestrogens, e.g. genistein and daidzein. An example of a synthetic steroidal estrogen is ethinyl estradiol, whereas tamoxifen and raloxifene are examples of synthetic non-steroidal estrogens. Thus, in summary, an estrogen as used herein may be selected from the group comprising natural steroidal estrogens; natural non-steroidal estrogens; synthetic steroidal estrogens; synthetic non-steroidal estrogens and estrogen-receptor agonists (selective α- and/or β-estrogen-receptor agonists). In preferred embodiments thereof, the estrogen is a natural estrogen, steroidal or non-steroidal. Of interest is any natural steroidal estrogen, for example such selected from the group comprising of 17-β-estradiol or esters thereof; estrone or esters thereof; estriol or esters thereof; and conjugated equine estrogens. The term "ester", in the present context, is denoted to mean any ester with an organic or inorganic acid. Typically, esters can be sulfates such as estrone sulfate, estriol sulfates or equilin sulfate. Furthermore, esters can be made with for example valeric acid, e.g to achieve 17-β-estradiol valerate. Esters may facilitate the absorption of an estrogen and after absorption the ester is split into the parent estrogenic compound.

As regards estrogen-receptor agonists it can be mentioned that a typical example on a α-selective estrogen-receptor agonist is 3,17β-dihydroxy-19-nor-17α-pregna-1,3,5(10)-trien-21,16α-lacton as disclosed in WO 02/26763. Typical examples on β-selective estrogen-receptor agonists are 8β-vinyl-estra-1,3,5(10),9(11)-tetraen-3,17β-diol, 8β-vinyl-estra-1,3,5(10)-trien-3,17β-diol, 8β-(2,2-difluorvinyl)-estra-1,3,5(10)-trien-3,17β-diol and 8β-methyl-estra-1,3,5(10)-trien-3,17β-diol as disclosed in WO 00/47603, WO 00/632228 and WO 01/77139.

Also the progestin may be of any kind, steroidal or non-steroidal, from a natural source or synthetically made. Generally, the progestin may be any without androgenic activity or at least without significant androgenic acitvity. Typically, the progestin may be selected from the group comprising chlormadinone, desogestrel, dienogest, progesterone, gestoden, levornorgestrel, medroxyprogesterone, cyproterone acetate, drospirenone, and derivatives thereof. However, it is anticipated that in some embodiments of the invention a progestin with androgenic activity may be advantageous. For example in the event where the female has polycystic ovary syndrom. Thus, cyproterone acetate or drospirenone may also be preferred progestins in embodiments of the invention.
In the present context, the term "derivatives thereof" is denoted to mean esters in the event where the progestin comprises a hydroxy group, such as acetates of for example chlormadinone and medroxyprogesterone. In presently preferred embodiments the progestin is levornorgestrel, cyproterone acetate or drospirenone.

Furthermore, in order to affect the absorption rate of the progestin and the estrogen, the hormones may be used in micronized form or as cydodextrin clathrates, or in any other physical or chemical modified form of the hormones that affects the dissolution rates in simulated gastric fluid and/or intestinal fluids in vitro or affects the bioavailability, such as affecting the absorption rate from the gastro-intestinal tract or affecting the degree of metabolism.

### Administration schemes

As stated *supra* the hormones should be administered in a suitable manner in order to duplicate physiological fluctuations of the estrogen and the progestin within the normal menstruation cycle of a fertile woman without inhibiting or suppressing ovulation.

Preferably, the hormones are administered orally, but other means of administering such as parenteral administration (e.g. intramuscular injections) or topical administration such as transdermally, by inhalation spray, rectally, nasally, buccally, vaginally or via an implanted reservoir are anticipated. The term parenteral as used herein includes transdermal, subcutaneous, intracutaneous, intravenous, intramuscular, intra-articular, intrasynovial, intrastemal, intrasternal, intrathecal, intralesional and intracranial injection or infusion techniques.
Thus, in general, the estrogen and/or the progestin may be administered in a form selected from the group comprising of per-oral administration, parenteral administration and/or topical administration.

Furthermore, the administering may be once daily, once every 2 days, once every 3 days or once a week or once every 4 weeks as long as the administration results in the delivery of daily doses of hormones that result in physiological relevant estrogen and/or progestin levels and fluctations in a female in her fertile age.

Should the half-life time of the hormone to be administered it require it is also possible that the daily dosage of the respective hormone is administered by multiple administration of accordingly subdivided dosages, e. g. by administering half of the dosage twice or a third of the dosage three times per day.

As stated, uses and methods of the invention implies the use of a pharmaceutical kit or a suitable formulation of a combination of the at least one estrogen and the at least one progestin. Suitably, the medicament is in a formulation selected from the group comprising oral formulation such as solid dosage forms as tablets, pills, capsules or granules, parenteral formulations such as injection solutions or topical formulations such as plasters, patches suppositories, creams, ointments and the like. Preferably, the medicament is in a formulation for oral administration that allows for systemic uptake of the hormones from the gastro-intestinal tract.

In general, methods and uses of the invention include the administration of and/or the delivery of a daily dose of the at least one estrogen for 15 to 28 days within each treatment period. Preferably, an estrogen is administered and/or delivered throughout a period of 18 to 28 days or 20 days to 27 days within each treatment period.

With regard to the at least one progestin, the methods and uses of the invention generally implies the administering and/or delivering of a daily dose of the at least one progestin throughout a period of from 10 to 28 days within each treatment period. Preferably, the progestin is administered and/or delivered throughout a period of 12 days to 25 days, more preferably throughout a period of 13 to 23 days, most preferably throughout a period of 14 to 22 days, such as administered for 20, 21 or 22 days within each treatment period.

Moreover, the method and uses of the invention generally include that the progestin is concurrently and/or simultaneously administered with the estrogen. Thus, typical embodiments of the invention comprises administering and/or delivering a daily dose of the at least one estrogen and a daily dose of the at least one progestin for 10 days to 28 days. Preferably, both the estrogen and the progestin is administered and/or delivered throughout a period of 12 days to 25 days, more preferably throughout a period of 13 to 23 days, most preferably throughout a period of 14 to 22 days, such as administered for 20, 21 or 22 days during each treatment period.

However, for some days, within each treatment period, it is suitable that the estrogen is administered or delivered as the only hormone. That is to say that no daily dose of the progestin is co-administered or co-delivered. Therefore, still typical embodiments of the invention include the administering of and/or delivering of a daily dose of the at least one estrogen for 1 to 21 days within each treatment period. In order to properly adjusting the hormones to physiological relevant hormone fluctuations, the estrogen is preferably administered and/or delivered for 7 to 14 days within each treatment period, wherein no daily dose of the progestin is co-administered or delivered.

In more specific terms, the methods and uses of the invention include at least two different phases of hormone administration, namely a first phase with the administering of estrogen followed by a second phase of administering a combination of an estrogen and a progestin. Thus, interesting embodiments of the invention comprises at least two phases within each treatment period, wherein
- a phase I comprises administering and/or delivering a daily dose of the at least one estrogen for at most 21 days; followed by
- a phase II comprises administering and/or delivering a daily dose of the at least one estrogen and the at least one progestin for 2 to 34 days.

In still more interesting embodiments thereof, phase I comprises administering and/or delivering a daily dose of the at least one estrogen for 4 to 21 days, more preferably for 6 to 14 days, even more preferably for 6 to 8 days, most preferably for 7 days. In presently interesting embodiments, phase I comprises administering and/or delivering a daily dose of the at least one estrogen for 6, 7 or 8 days.

In still further embodiments thereof, phase II comprises administering a daily dose of the at least one estrogen and the at least one progestin for 5 to 28 days, more preferably for 8 to 22 days, most preferably for 14 days. In presently interesting embodiments, phase II comprises administering a daily dose of the at least one estrogen and the at least one progestin for 13, 14 or 15 days within each treatment period.

In some embodiments of the invention, phase II is further divided into two sub-phases for the purpose of changing the dose of progestin from the first sub-phase to the next sub-phase. Therefore, in some methods and uses of the invention phase II is divided into two sub-phases IIa and IIb. In further embodiments thereof, sub-phase IIb comprises administering a proportionally higher daily dose of the at least one progestin relatively to the dose administered in sub-phase IIa. In such embodiments, wherein phase II is divided into two sub-phases, the proportionally higher daily dose of the at least one progestin of said sub-phase IIb relatively to the dose of sub-phase IIa is in a weighed or molar ratio ranging from about 1.1:1 to 4:1, preferably from about 1.5:1 to 3:1, most preferably from about 1.7:1 to 2.5:1, such as 2:1. The sub-phases may have different time length.

In each dosage unit containing a progestin or a progestin and an estrogen the progestin is present in an amount which does not inhibit ovulation.

Furthermore, each treatment period may include a further phase wherein none of the said hormones are administered or delivered. Thus, in still suitable embodiments thereof, a further phase (herein named phase III) comprises the administering of a pharmaceutically acceptable placebo or no administration of an estrogen and a progestin for at most 21 days within each treatment period. Preferably, phase III comprises 4 to 21 days, more preferably 6 to 14 days, most preferably 7 days. It is envisaged that phase III may eventually comprises the administering of low doses of an estrogen.

A treatment period may optionally comprise a further phase immediately following the second phase. Thus, in still further embodiments of the above, the method and uses of the invention further comprises a phase IIE comprising administering and/or delivering a daily dose of the at least one estrogen for 1 day. However, in presently interesting embodiments, phase IIE is not included in the treatment period.

In typical embodiments of the invention, phase I comprises administering and/or delivering a daily dose of the at least one estrogen for 7 days, phase IIa comprises administering and/or delivering a daily dose of the at least one estrogen and the at least one progestin for 7 days and phase IIb comprises administering and/or delivering a daily dose of the at least one estrogen and the at least one progestin for 7 days. In addition, phase III may comprise administering a placebo, no estrogen and no progestin for 7 days.

As may be understood, the methods and uses of the present invention implies that phase I, phase II and phase III is in chronological order. Furthermore, sub-phase IIa and sub-phase IIb is preferably in chronological order.

As stated an aspect of the invention relates to the use of a combination of at least one estrogen and at least one progestin for the preparation of a medicament for increasing likelihood of conception in a female, for the treatment of low fecundity in a female and/or for providing menstrual cycle control in a female without suppressing ovulation. Such a medicament is preferably in the form of a number of dosage units intended for use in a treatment period of 21 to 35 days such that at least one estrogen is present in 14 to 34 of said dosage units. Moreover, the medicament comprises, for a treatment period of 21 to 35 days, 2 to 34 of dosage units comprising at least one progestin. In another form, the medicament is in the form of a number of dosage units intended for use in a treatment period of 21 to 35 days, and wherein the at least one estrogen and the at least one progestin is both present in 2 to 34 of said dosage units.

The medicament may further comprises up to 21 progestin-free dosage units comprising the at least one estrogen, preferably 4 to 21, more preferably 6 to 14 progestin-free dosage units.

In some embodiments, the medicament comprises up to 21 dosage units, preferably 6 to 14 dosage units comprising per dosage unit the at least one estrogen in a dose therapeutically equivalent to 17-β-estradiol from about 2.5 to 5 mg. In further embodiments, the medicament comprises 2 to 34 dosage units, preferably 5 to 28, more preferably from 8 to 22, most preferably 14 dosage units comprising per dosage unit the at least one estrogen in a dose therapeutically equivalent to 17-β-estradiol from about 1.5 to 2.4 mg.

As may be understood, the medicament may comprise a pharmaceutical kit as described herein.

### Pharmaceutical kit

As stated previously, methods and uses of the invention may include the use of a pharmaceutical kit comprising
- a first section comprises at most 21 dosage units of at least one estrogen; and
- a second section comprises from 2 to 34 dosage units of at least one estrogen and from 2 to 34 dosage units of at least one progestin or comprises from 2 to 34 dosage units of at least one estrogen and at least one progestin provided that in each dosage unit containing a progestin or a progestin and an estrogen the progestin is in a non-ovulation inhibiting amount; and
- optionally a third section comprises at most 21 dosages of a pharmaceutically acceptable placebo or a blank.

In suitable embodiments thereof, the number of dosage units in the first section amounts to 4 to 21, preferably 6 to 14, more preferably 6 to 8 and most preferably to 7.

Furthermore, in still further embodiments, the number of dosage units in the second section amounts from 5 to 28, preferably from 8 to 22 and most preferably to 14. In presently interesting embodiments the number of dosage units in the second section amounts to 13, 14, or 15. In typical embodiments thereof the second section is divided into two sub-sections, IIa and IIb. In further embodiments thereof the dosage units of the sub-section IIb comprises a proportional higher amount of the at least one progestin relatively to dosage units of the sub-section IIa. Notably, the proportional higher amount of the progestin in dosage units of sub-section IIb relatively to dosage units of the sub-section IIa is in a weighed or molar ratio in the range from about 1.1:1 to 10:1, preferably from about 1.2:1 to 5:1, even more preferably from about 1.5 to 4:1, most preferably from about 1.7:1 to 2.5:1, such as 2:1.

Suitable, the number of dosage units in sub-section IIa amounts to 1 to 21 dosage units, preferably from 4 to 21, more preferably to 6 to 14, most preferably to 7. The number of dosage units in sub-section IIb ranges from 1 to 21, preferably from 4 to 21, more preferably from 6 to 14, most preferably 7. In presently interesting embodiments, the number of dosage units in IIa and IIb is each 6, 7 or 8.

In still further embodiments thereof, the number of units in the third section amounts to 4 to 21, preferably 6 to 14, more preferably to 6 to 8, most preferably to 7.

In presently interesting embodiments of the invention, the number of dosage units in sub-section IIa is 7 and the number of dosage units in sub-section IIb is 7. In further interesting embodiments thereof, section I comprises 7 dosage units.

The kit comprises optionally a further section placed between the second and third section. This section, named IIE comprises of one dosage unit comprising at least one estrogen but no progestin. Thus, in some embodiments of the invention a section IIE is not present or a placebo will replace it.

### Doses

According to the invention, the methods, uses and kits generally include that the at least one estrogen to be administered or comprised in a dosage unit is in a dose therapeutically equivalent to 17-β-estradiol of from about 0.1 mg to 10 mg depending on the nature of the estrogen. Thus, in embodiments of the invention the daily dose of the at least one estrogen to be administered and/or delivered in phase I is in a daily dose therapeutically equivalent to 17-β-estradiol of from about 0.1 mg to 10 mg. Likewise, each dosage unit of the first section of the pharmaceutical kit comprises the at least one estrogen in a dose therapeutically equivalent to 17-β-estradiol of from about 0.1 mg to 10 mg. Preferably, the dose is therapeutically equivalent to 0.5 to 5 mg of 17-β-estradiol, more preferably therapeutically equivalent to about 1 to 4 mg, more preferably therapeutically equivalent to about 2 to 4 mg, most preferably therapeutically equivalent to 4 mg of mg of 17-β-estradiol.

In phase II of the present invention, the at least one estrogen is in an amount therapeutically equivalent to 17-β-estradiol of from about 0.1 mg to 10 mg depending on the estrogen. Likewise, each dosage unit of the second section of the kit comprises a dose of the at least one estrogen in an amount therapeutically equivalent to 17-β-estradiol from about 0.1 mg to 10 mg. Preferably, the dose is therapeutically equivalent to 0.2 to 5 mg of 17-β-estradiol, more preferably therapeutically equivalent to about 0.5 to 3 mg, even more preferably therapeutically equivalent to about 1 to 3 mg, most preferably therapeutically equivalent to 2 mg of mg of 17-β-estradiol.

In general, the daily dose of the at least one progestin should be lower than the ovulation inhibition dose. In embodiments where the progesting is levonorgestrel the daily dose to be adminstered or delivered to the systemic circulation is such that resulting serum blood levels will be about from 2000 pg/ml to 6000 pg/ml serum. Thus, in some embodiments, the progestin in said phase II is in an amount therapeutically equivalent to levornorgestrel from about 2 µg to 40 µg. Correspondingly, each dosage unit of said second section of the kit comprises a dose of the at least one progestin in a therapeutically equivalent dose of levornorgestrel from about 2 µg to 40 µg. Preferably, the dose is therapeutically equivalent to a dose of levornorgestrel from about 5 to 30 µg, more preferably from about 10 to 25 µg.

In sub-phase IIa, the at least one progestin is in a daily dose therapeutically equivalent to a dose of levornorgestrel from about 2 µg to 30 µg. Thus, each dosage unit of said sub-section IIa comprises a dose of the at least one progestin in a dose therapeutically equivalent to a dose of levornorgestrel from about 2 µg to 30 µg, Preferably, the dose is therapeutically equivalent to a dose of levornorgestrel from about 5 to 20 µg, more preferably from about 7.5 to 15 µg.

In sub-phase IIb the at least one progestin is in a daily dose therapeutically equivalent to about 5 µg to 40µg of levornorgestrel. Correspondingly, each dosage unit of said sub-section IIb comprises a dose of the at least one progestin in an amount therapeutically equivalent to a dose of levornorgestrel from about 5 µg to 40 µg. Preferably, the dose is therapeutically equivalent to about 5 to 30 µg, more preferably from about 10 to 25 µg.

As the skilled artisan will appreciate, lower or higher doses than those recited above may be required. Specific dosage and treatment regiments for any particular patient will depend upon a variety of factors, including the activity of the specific compound employed, the age, body weight, general health status, diet, time of administration, rate of excretion and hormone combination.
The ovulation inhibition doses of different progestins have been well defined in clinical studies. Doses of at least 50% below this dose are are believed not to inhibit ovulation.

The progestagenic potency of progestins have been defined clinically in humans by the endometrial transformation dose and the ovulation inhibtion dose. These data are available in the literature.

Estrogenic potencies are defined pre-clinically by the uterotropic activity and clinically in humans by assessing vaginal cornification (maturation value). These data are available in the literature.

### EXAMPLES

### Example 1

A typical dosing regimen comprises the following administration scheme of an estrogen and a progestin:

| Phase | Hormone | Dose | Days of treatment |
|---|---|---|---|
| I | Estradiol | 4 mg | 7 |
| Iia | Estradiol + Levornorgestrel | 2 mg 10µg | 7 |
| Iib | Estradiol + Levornorgestrel | 2 mg 20µg | 7 |
| III | Placebo | | 7 |

Women with desire to get pregnant and who do not want to be treated in an infertilty center visit a gynecological practice. Andrologic causes of infertility of the partner have to be excluded elsewhere in advance. After a thorough anamnesis, the gynecologist performs a clinical examination in order to exclude anatomic abnormalities and tubal obstruction. The women starts pill-intake of the kit at her first day of the next menstruation. Instructions about intercourse frequencies are provided. In case of missing menstruation, a pregnancy test has to be performed. ß-HCG in serum is the primary target parameter in a clinical study.

## Claims

1. A pharmaceutical kit comprising 21 to 35 dosage units or a multiple of 21 to 35 dosage units, wherein within each multiple of 21 to 35 dosage units
- a first section comprises at most 21 dosage units of at least one estrogen; and
- a second section comprises from 2 to 34 dosage units of at least one estrogen and from 2 to 34 dosage units of at least one progestin or comprises from 2 to 34 dosage units of at least one estrogen and at least one progestin provided that in each dosage unit containing a progestin or a progestin and an estrogen the progestin is in a non-ovulation inhibiting amount; and
- optionally a third section comprises at most 21 dosages of a pharmaceutically acceptable placebo or a blank.

2. The kit according to claim 1, wherein the number of dosage units in the first section amounts to 4 to 21, preferably 6 to 14, more preferably 6 to 8 and most preferably to 7.

3. The kit according to any one of claims 1 or 2, wherein the number of dosage units in the second section amounts to 5 to 28, preferably 8 to 22 and most preferably to 14.

4. The kit according to any one of preceding claims, wherein the number of dosage units in the third section amounts to 4 to 21, more preferably 6 to 14 and most preferably to 7.

5. The kit according to any one of preceding claims, wherein the second section is divided into two sub-sections, IIa and IIb, such that the dosage units of sub-section IIb comprises a proportional higher amount of the at least one progestin relatively to that in dosage units of the sub-section IIa.

6. The kit according to claim 5, wherein the number of dosage units in sub-section IIa amounts to 1 to 21 dosage units, preferably 4 to 21 dosage units, more preferably 6 to 14 dosage units, most preferably 7.

7. The kit according to claim 5, wherein the number of dosage units in sub-section IIb amounts to 1 to 21, preferably 4 to 21, more preferably 6 to 14 and most preferably 7.

8. The kit according to any one of claims 5 to 7, wherein the proportional higher amount in said dosage units of sub-section IIb relatively to that of said dosage units of the sub-section IIa is in a weighed ratio in the range from about 1.1:1 to 10:1, preferably from about 1.2:1 to 5:1, even more preferably from about 1.5 to 4:1, most preferably from about 1.7:1 to 2.5:1, such as 2:1 provided that the amount of the at least one progestin is a non-ovulation inhibiting amount in each dosage unit of sub-sections Iia and IIb.

9. The kit according to any one of preceding claims, wherein the at least one estrogen is selected from the group consisting of natural steroidal estrogens; natural non-steroidal estrogens; synthetic steroidal estrogens; synthetic non-steroidal estrogens and selective estrogen-receptor α- and β-agonists.

10. The kit according to any one of preceding claims, wherein the at least one estrogen is a natural steroidal estrogen or a natural non-steroidal estrogen.

11. The kit according to any one of claims 9 or 10, wherein the natural steroidal estrogens is selected from the group consisting of 17-β-estradiol or esters thereof; estrone or esters thereof; estriol or esters thereof; and conjugated equine estrogens.

12. The kit according to claim 11, wherein the at least one estrogen is 17-β-estradiol.

13. The kit according to any one of preceding claims, wherein the at least one progestin is a steroidal or non-steroidal progestin.

14. The kit according to any one of preceding claims, wherein the at least one progestin is selected from the group consisting of chlormadinone, cyproterone acetate, desogestrel, dienogest, drospirenone, gestoden, levornorgestrel, medroxyprogesterone, progesterone and derivatives thereof.

15. The kit according to any one of preceding claims, wherein the at least one progestin is selected from the group consisting of cyproterone acetate, drospirenone, levornorgestrel, dienogest and derivatives thereof.

16. The kit according to any one of preceding claims, wherein each dosage unit of said first section comprises the at least one estrogen in a dose therapeutically equivalent to a dose of 17-β-estradiol from about 0.1 mg to 10 mg, preferably from about 0.5 to 5 mg, more preferably from about 1 to 4 mg, even more preferably from about 2 to 4 mg, most preferably 4 mg.

17. The kit according to any one of preceding claims, wherein each dosage unit of said second section comprises the at least one estrogen in a dose therapeutically equivalent to a dose of 17-β-estradiol from about 0.1 mg to 10 mg, preferably from about 0.2 to 5 mg, more preferably from about 0.5 mg to 3 mg, even more preferably from about 1 to 3 mg, most preferably 2 mg.

18. The kit according to any one of preceding claims, wherein each dosage unit of said second section comprises the at least one progestin in a dose therapeutically equivalent to levornorgestrel from about 2 µg to 40 µg, preferably from about 5 to 30 µg, more preferably from about 10 to 25 µg.

19. The kit according to any one of claims 5 to 18, wherein each dosage unit of said sub-section IIa comprises the at least one progestin in a dose therapeutically equivalent to a dose of levornorgestrel from about 2 µg to 30 µg, preferably from about 5 to 20 µg, more preferably from about 7.5 to 15 µg.

20. The kit according to any one of claims 5 to 18, wherein each dosage unit of said sub-section IIb comprises the at least one progestin in a dose therapeutically equivalent to a dose of levornorgestrel from about 5 µg to 40 µg, preferably from about 5 to 30 µg, more preferably from about 10 to 25 µg.

21. Use of a combination of at least one estrogen and at least one progestin for the preparation of a medicament for providing menstrual cycle control in a female without suppressing ovulation.

22. Use of a combination of at least one estrogen and at least one progestin for the preparation of a medicament for increasing likelihood of conception in a female.

23. Use of a combination of at least one estrogen and at least one progestin for the preparation of a medicament for the treatment of low fecundity in a female.

24. The use according to any one of claims 21 to 23, wherein said female has experienced menses at least once.

25. The use according to any one of claims 21 to 24, wherein said female is in the age ranging from about 18 to 60 years, preferably ranging from about 25 to 55 years, more preferably ranging from 30 to 50 years, most preferably ranging from 35 to 50 years.

26. The use according to any one of claims 21 to 24, wherein the female is in a low fecundity category selected from the group consisting of a female over 30 years of age; a female previously on the birth control pills; a female with irregular menstrual cycle.

27. The use according to any one of claims 21 to 26, wherein the at least one estrogen is selected from the group consisting of natural steroidal estrogens; natural non-steroidal estrogens; synthetic steroidal estrogens; synthetic non-steroidal estrogens and selective estrogen-receptor α- and β-agonists.

28. The use according to any one of claims 21 to 27, wherein the at least one estrogen is a natural steroidal estrogen or a natural non-steroidal estrogen.

29. The use according to any one of claims 27 or 28, wherein the natural steroidal estrogens is selected from the group consisting of 17-β-estradiol or esters thereof; estrone or esters thereof; estriol or esters thereof; and conjugated equine estrogens.

30. The use according to claim 29, wherein the at least one estrogen is 17-β-estradiol.

31. The use according to any one of claims 21 to 30, wherein the at least one progestin is a steroidal or non-steroidal progestin.

32. The use according to claim 31, wherein the at least one progestin is selected from the group consisting of chlormadinone, cyproterone acetate, desogestrel, dienogest, drospirenone, gestoden, levornorgestrel, medroxyprogesterone, progesterone and derivatives thereof.

33. The use according to claim 32, wherein the at least one progestin is selected from the group consisting of cyproterone acetate, drospirenone, levornorgestrel, dienogest and derivatives thereof.

34. The use according to any one of claims 21 to 33, wherein the medicament is in the form of a number of dosage units intended for use in a treatment period of 21 to 35 days, and wherein the at least one estrogen is present in 14 to 34 of said dosage units.

35. The use according to any one of claims 21 to 34, wherein the medicament is in the form of a number of dosage units intended for use in a treatment period of 21 to 35 days, and wherein the at least one progestin is present in 2 to 34 of said dosage units.

36. The use according to any one of claims 21 to 34, wherein the medicament is in the form of a number of dosage units intended for use in a treatment period of 21 to 35 days, and wherein the at least one estrogen and the at least one progestin is both present in 2 to 34 of said dosage units.

37. The use according to any one of claims 35 or 36, wherein the medicament further comprises up to 21 progestin-free dosage units comprising the at least one estrogen, preferably 4 to 21, more preferably 6 to 14, even more preferably 7 progestin-free dosage units comprising the at least one estrogen.

38. The use according to any one of claims 21 to 37, wherein the at least one estrogen is in a dose therapeutically equivalent to 17-β-estradiol from about 0.1 mg to 10 mg, preferably from about 0.5 to 5 mg, more preferably from about 1 to 4 mg, most preferably 4 mg.

39. The use according to any one of claims 21 to 38, wherein the at least one progestin is in a dose therapeutically equivalent to levornorgestrel from about 2 mg to 40 µg, preferably from about 5 to 30 µg, more preferably from about 10 to 25 µg.

40. The use according to any one of claims 31 to 39, wherein the medicament comprises
up to 21 dosage units comprising the at least one estrogen in a dose therapeutically equivalent to 17-β-estradiol valerate from about 2.5 to 5 mg.

41. The use according to any one of claims 34 to 40, wherein the medicament comprises 2 to 34 dosage units, preferably 5 to 28, more preferably from 8 to 22, most preferably 14 dosage units comprising the at least one estrogen in a dose therapeutically equivalent to 17-β-estradiol valerate from about 1.5 to 2.4 mg.

42. The use according to any one of claims 34 to 41, wherein the medicament comprises
at most 3 dosage units comprising the at least one estrogen in a dose therapeutically equivalent to 17-β-estradiol valerate from about 0.5 to 1.4 mg.

43. The use according to any one of claims 34 to 42, wherein the medicament comprises
1 to 7 dosage units, preferably from 3 to 5 dosage units, most preferably 3 or 4 dosage units comprising the at least one progestin in a dose therapeutically equivalent to levornorgestrel from about 2 to 15 µg.

44. The use according to any one of claims 34 to 43, wherein the medicament comprises
7 to 21 dosage units, preferably from 7 to 19 dosage units, more preferably from 7 to 18 dosage units comprising the at least one progestin in a dose therapeutically equivalent to levornorgestrel from about 10 to 50 µg.

45. The use according to any one of claims 21 to 44, wherein the medicament is in a formulation selected from the group consisting of oral formulation, parenteral formulation and topical administration.

46. The use according to claim 45, wherein the medicament is in an oral formulation.

47. The use according to claim 45, wherein the medicament is at least partially in a transdermal formulation.

48. The use according to any one of claims 21 to 47, wherein said medicament is a kit as defined by any one of claims 1 to 20.

49. A method for providing menstrual cycle control in a female without suppressing ovulation comprising administering to a female, in a treatment period of at least 21 days to 35 days or multiple periods thereof, an effective dose of at least one estrogen and at least one progestin.

50. The method according to claim 49 for increasing likelihood of conception in a female comprising administering to a female, in a treatment period of at least 21days to 35 days or multiple periods thereof, an effective dose of at least one estrogen and at least one progestin.

51. The method according to claim 49, for treatment of low fecundity in a female.

52. The method according to claim 49, wherein said female has experienced menses at least once.

53. The method according to claim 49, wherein said female is in the age ranging from about 18 to 60 years, preferably ranging from about 25 to 55 years, more preferably ranging from 30 to 50 years, most preferably ranging from 35 to 50 years.

54. The method according to claim 49, wherein the female is in a low fecundity category selected from the group consisting of a female over 30 years of age; a female previously on the birth control pills; a female with irregular menstrual cycle.

55. The method according to claim 49, wherein the at least one estrogen is selected from the group consisting of natural steroidal estrogens; natural non-steroidal estrogens; synthetic steroidal estrogens; synthetic non-steroidal estrogens and selective estrogen-receptor α- and β-agonists.

56. The method according to claim 49, wherein the at least one estrogen is a natural steroidal estrogen or a natural non-steroidal estrogen.

57. The method according to claim 49, wherein the natural steroidal estrogens is selected from the group consisting of 17-β-estradiol or esters thereof; estrone or ) esters thereof; estriol or esters thereof; and conjugated equine estrogens.

58. The method according to claim 49, wherein the at least one estrogen is 17-β-estradiol.

59. The method according to claim 49, wherein the at least one progestin is a steroidal or non-steroidal progestin.

60. The method according to claim 49, wherein the at least one progestin is selected from the group consisting of chlormadinone, cyproterone acetate, desogestrel, dienogest, drospirenone, gestoden, levornorgestrel, medroxyprogesterone, progesterone and derivatives thereof.

61. The method according to claim 49, wherein the at least one progestin is selected from the group consisting of cyproterone acetate, drospirenone, levornorgestrel, dienogest and derivatives thereof.

62. The method according to claim 49, comprising, within each treatment period, administering a daily dose of the at least one estrogen for 15 to 28 days, preferably for 18 to 28 days, most preferably for 20 days to 27 days.

63. The method according to claim 49, comprising, within each treatment period, administering a daily dose of the at least one progestin for 10 to 28 days, preferably for 12 days to 25 days, more preferably for 13 to 23 days, most preferably for 14 to 22 days.

64. The method according to claim 49, comprising, within each treatment period, administering a daily dose of the at least one estrogen and a daily dose of the at least one progestin for 10 days to 28 days, preferably for 12 to 25 days, more preferably for 13 to 23 days, most preferably for 14 to 22 days.

65. The method according to claim 49, comprising, within each treatment period, administering a daily dose of the at least one estrogen for 1 to 8 days, preferably from 2 to 7 days, more preferably from 2 to 5 days, even more preferably from 2 to 4 days, wherein no daily dose of the progestin is co-administered.

66. The method according to claim 49, comprising, within each treatment period, at least two phases:
- a phase I comprising administering a daily dose of at least one estrogen for at most 21 days, preferably for 4 to 21 days, more preferably for 6 to 14 days, even more preferably for 6 to 10 days, most preferably for 7days; followed by
- a phase II comprising administering a daily dose of at least one estrogen and at least one progestin for 2 to 34 days, preferably for 5 to 28 days, more preferably for 8 to 22 days, most preferably for 14 days.

67. The method according to claim 66, further comprising a phase III comprising administering a pharmaceutically acceptable placebo for at most 21 days, preferably for 4 to 21 days, more preferably for 6 to 14 days, most preferably for 7 days.

68. The method according to claim 66, further comprising a phase III wherein the estrogen and progestin is not administered for at most 21 days, preferably for 4 to 21 days, more preferably for 6 t 14 days, most preferably for 7 days.

69. The method according to claim 66, wherein said phase II comprises two sub-phases, IIa and IIb, said sub-phase IIb comprises administering a proportionally higher daily dose of the at least one progestin relatively to the dose administered in sub-phase IIa.

70. The method according to claim 69, wherein the proportionally higher daily dose of the at least one progestin of said sub-phase IIb relatively to the dose of sub-phase IIa is in a weighed ratio ranging from about 1.1:1 to 4:1, preferably from about 1.5:1 to 3:1, most preferably from about 1.7:1 to 2.5:1, such as 2:1.

71. The method according to claim 69, wherein the sub-phase IIa is in a time length of from about 1 to 21 days, preferably from about 4 to 21 days, more preferably from about 6 to 14 days and most preferably 7 days.

72. The method according to claim 69, wherein sub-phase IIb is in a time length of from about 1 to 21 days, preferably from about 4 to 21 days, more preferably from about 6 to 14 days and most preferably 7 days.

73. The method according to claim 66, wherein the daily dose of the at least one estrogen in said phase I is in an amount from about 0.1 mg to 10 mg, preferably from about 0.5 to 5 mg, more preferably from about 1 to 4 mg, even more preferably from about 2 to 4 mg, most preferably 4 mg of 17β-estradiol or another estrogen in an amount therapeutically equivalent to the aforementioned doses of 17β-estradiol.

74. The method according to claim 66, wherein the daily dose of the at least one estrogen in said phase II is in an amount from about 0.1 mg to 10 mg, preferably from about 0.2 to 5 mg, more preferably from about 0.5 mg to 3 mg, even more preferably from about 1 to 3 mg, most preferably 2 mg of 17β-estradiol or another estrogen in an amount therapeutically equivalent to the aforementioned doses of 17β-estradiol.

75. The method according to claim 66, wherein the daily dose of the at least one progestin in said phase II is in a dose therapeutically equivalent to levornorgestrel from about 2 µg to 40 µg, preferably from about 5 to 30 µg, more preferably from about 10 to 25 µg.

76. The method according to claim 69, wherein the daily dose of the at least one progestin in said sub-phase IIa is in a dose of therapeutically equivalent to a dose of levornorgestrel from about 2 µg to 30 µg, preferably from about 5 to 20 µg, more preferably from about 7.5 to 15 µg.

77. The method according to claim 69, wherein the daily dose of the at least one progestin in said sub-phase IIb is in a dose therapeutically equivalent to a dose of levornorgestrel from about 5 µg to 40 µg, preferably from about 5 to 30 µg, more preferably from about 10 to 25 µg.

78. The method according to claim 65, wherein said phases: phase I, phase II and phase III is in chronological order.

79. The method according to claim 69, wherein said phases: sub-phase IIa and sub-phase IIb is in chronological order.

80. The method according to claim 49, wherein the at least one estrogen and the at least one progestin is administered in a form selected from the group consisting of oral administration, parenteral administration and topical administration.

81. The method according to claim 80, wherein the at least one estrogen and/or the at least one progestin is administered by oral administration.

82. The method according to claim 80, wherein the at least one estrogen and/or the at least one progestin is administered at least partially by transdermal administration.

83. The method according to claims 49, wherein the administering is once daily, once every 2 days, once every 3 days or once week.

84. The method according to claim 49, wherein the administration is twice daily or three times daily by subdividing the respective daily doses.
